# EUROPEAN PATENT APPLICATION

(11) **EP 1 484 390 A1**
(43) Date of publication of application: **08.12.2004**
(21) Application number: 03012551.2
(22) Date of filing: 02.06.2003
(51) Int. Cl.: C12N 5/06, B01D 39/06, B01D 39/08, B01D 39/20, A61M 1/36

(54) **Methods of preparing peripheral stem cells from leukocyte reduction filters**

(71) Applicant: Blutspendedienst des Bayerischen Roten Kreuzes, 80336 München (DE)
(72) Inventor: Illert, Werner, 97072 Würzburg (DE)
(74) Representative: Schohe, Stefan

(57) **Abstract**

The present invention relates to a method of preparing a peripheral blood stem cell enriched cell fraction from a used leukocyte reduction filter comprising the steps of a) back-flushing a leukocyte reduction filter, which has been used for the preparation of leukocyte poor blood, with a buffer comprising a chelating compound, preferably ethylenediaminetetraacetic acid (EDTA) and a disaccharide, preferably saccharose and b) recovering the cells as well as the use of leukocyte reduction filters for the preparation of a cell suspension enriched for peripheral blood stem cells.

## Description

The present invention relates to a method of preparing a peripheral blood stem cell enriched cell fraction from a used leukocyte reduction filter comprising the steps of a) back-flushing a leukocyte reduction filter, which has been used for the preparation of leukocyte poor blood, with a buffer comprising a chelating compound, preferably ethylenediaminetetraacetic acid (EDTA) and a disaccharide, preferably saccharose and b) recovering the cells as well as the use of leukocyte reduction filters for the preparation of a cell suspension enriched for peripheral blood stem cells.

Peripheral blood stem cells are a sub population of leukocytes, which are present in very small concentrations in peripheral blood. These cells are also called hematopoetic precursor cells and are characterized by their potency to form any other hematopoetic cell lineage a characteristic also called pluripotency (Punzel M, Ho AD: Divisional history and pluripotency of human hematopoietic stem cells. Ann.N.Y.Acad.Sci. 2001 (938):72-81). Stem cells have been found to express the CD34 cells surface antigen, which is therefore taken as a marker for stem cells. Because of its ability to regenerate the entire hematopoetic system the use of stem cells has been proposed for a variety of different therapeutical applications including, for example, reconstitution of the hematopoetic system after chemo or radiation therapy, provision of a new hematopoetic system in anemic patients like, for example, patients suffering of Fanconi anemia and/or treatment of patients having other blood diseases, e.g., sickle cell anemia. Other applications are in treatment of breast cancer, myeloma or lymphoma (Shpall EJ, Jones RB, Bearman SI, et al.: Transplantation of CD34-positive autologous marrow into breast cancer patients following high-dose chemotherapy: Influence of CD34-positive peripheral blood progenitors and growth factors on engraftment. J. Clin. Oncol. 1994 (12):28-36; Mahe B, Milpied N, Hermouet S et al.: G-CSF alone mobilizes sufficient peripheral blood CD34+ cells for positive selection in newly diagnosed patients with myeloma and lymphoma. Br. J. Haematol. (92):263-268). Stem cells can be derived from a variety of sources including, bone marrow (Kotton DN, Ma BY et al.: Bone marrow derived cells as progenitors of lung alveolar epithelium. Development (128):5181, Yuehna J, Verfaillie CM: Pluripotency of mesenchymal stem cells derived from adult marrow. Nature 2002 (418):41-49), cord blood (Lu L, Shen RN, Broxmeyer HE: Stem cells from bone marrow, umbilical cord blood and peripheral blood for clinical application: current status and future application. Crit. Rev. Oncol. Hematol. 1996 (22):61-78) or can be derived from circulating blood cells, i.e. from the peripheral blood (Platzecker U, Prange-Krex G, Bornhäuser M et al.: Spleen enlargement in healthy donors during G-CSF mobilization ofPBPCs: Transfusion 2001 (41):184-189).

However, the recovery of stem cells from bone marrow is difficult and involves the surgical removal of bone marrow with a syringe from a bone. This process is associated with a high level of discomfort for the donor. A few years ago the recovery of stem cells from cord blood of newborns has been reported and has become more widespread, however, it is only feasible if the pregnancy is carried until late term and even then the amount of cells obtained from the procedure is only sufficient for later therapeutical applications in 50 percent of the cases. Furthermore stem cells have also been obtained from the peripheral blood system. Since the number of circulating stem cells is very low this method is only feasible, if the stem cells are induced to leave their normal location in the bone marrow, i.e. if they are "mobilized". Mobilization is achieved with haemapoetic growth factors like, for example, granulocyte-colony stimulating factor (G-CSF). Once the stem cells are mobilized they can be harvested from the peripheral blood by, for example, cytapherese. However, the stimulation has recently shown to be associated with an enlargement of the spleen and, thus, albeit less invasive for the donor is also associated with a risk (U. Platzbecker et al. (2001) Transfusion 41:184-189). Furthermore the treatment with recombinant G-CSF is very expensive.

Without stimulation peripheral blood contains only between one and ten stem cells per µl. One µl whole blood contains about five million blood cells in total, i.e. stem cells are contained in whole blood at a concentration of 0.0002 to 0.002%. Thus, given the numerous medical uses of stem cells and the lack of a safe, cost-effective and easy way to obtain those cells new methods to obtain blood stem cells are needed.

The present invention is based on the surprising finding that a cell population enriched for peripheral blood stem cells can be obtained from used leukocyte reduction filters. These filters are routinely used to remove leukocytes from cellular blood products like erythrocyte and thrombocyte concentrates, which are subsequently used for transfusion. Leukocytes are removed prior to use since they can lead to undesired side effects in patients. The side effects reach from mild HLA-induced incompatibilities to severe life threatening infections due to intracellular microorganisms. Furthermore leukocytes contained within the blood products can upon disintegration release components enzymes and cytokines, which negatively effect the quality and storage life of the blood product. For these reasons a variety of different leukocyte reduction filters have been developed and the best are now capable of reducing the number of leukocytes in an erythrocyte concentrate by the factor of 10,000.

The regulatory health authorities in Europe (United Kingdom, France, Switzerland, Austria, Sweden Denmark, Norway, Portugal, Germany) and other countries (Canada, Australia) have reacted to the risks associated with leukocytes in blood products and have now stipulated that all blood used for blood transfusion has to be depleted of leukocytes by filtration. Thus, in the future large numbers of used leukocyte reduction filters will be generated and discarded in the blood processing industry. Therefore, J.-H. Weitcamp and J. E. Crowe, Jr. (2001) BioTechniques 31:464-466 have proposed to employ used leukocyte reduction filters for the isolation of a human B cell fraction. Upon application of a solution comprising PBS and 5 mM EDTA about 1.5 x 10⁸ peripheral blood mononuclears cells (PBMC) where obtained from each filter, which had previously been used to deplete 500 ml of blood. S. Ebner et al. (2001) J. Immunological Methods 252:93-104 disclosed the isolation of a human dendritic cell fraction from used leukocyte reduction filters. The buffer used for back-flushing comprised 9% dextran, M.W. 70,000, 2.5% saccharose and 3% human serum albumin in PBS. The recovered cell fraction was very similar to the so called "buffy-coat" fraction, which is a fraction derived from the interphase formed in blood, after separation into blood cells and plasma. The isolation of a stem cell enriched fraction from leukocyte reduction filters was not taught in any of those documents. S. Ebner et al. (supra), who were analyzing the cellular composition of its dendritic cells fraction using antibodies against several surface markers did not even look for the presence of CD34⁺ cells, which is not surprising given that those cells are known to be scarce and delicate and, thus, would not have been expected by someone of skill in the art to be recoverable from leukocyte reduction filters by back-flushing.

The present invention provides a method, which allows the recovery of a cell fraction enriched for peripheral blood stem cells. Thus, in one aspect the method of the present invention involves the elution of a peripheral blood stem cell enriched cell fraction from a used leukocyte reduction filter comprising the steps of: a) back-flushing a leukocyte reduction filter, which has been used for the preparation of leukocyte poor blood with a buffer comprising a chelating compound preferably EDTA (including salts thereof, in particular sodium and potassium salts) and a disaccharide preferably saccharose and b) recovering the cells.

The term "peripheral blood stem cell enriched cell fraction" refers to a blood cell derived cell fraction comprising at least about 0.01% CD34⁺ cells. Preferably, at least about 0.02%, more preferably at least about 0.03%, more preferably at least about 0.04%, more preferably at least about 0.05%, more preferably at least about 0.06%, more preferably at least about 0.07%, more preferably at least about 0.08%, more preferably at least about 0.09% and most preferably at least about 0.1 % CD34⁺ cells. Given the usual concentration range of 0.0002 to 0.002% of CD34⁺ cells in whole blood the enrichment achieved with the method of the present invention equates to a 10- to 500-fold enrichment. Since the amount of stem cells in the starting material, i.e. whole blood of a donor, varies markedly the enrichment factor also varies markedly. However, in a preferred embodiment the CD34⁺ cells are at least about 50-fold enriched, preferably at least about 100-fold and most preferably at least about 500-fold enriched in the cell fraction produced with the method of the present invention in comparison with the starting material.

The term "used leukocyte reduction filter" refers to a leukocyte reduction filter onto which blood, preferably whole blood, has been applied. Generally the blood is applied by gravity from the pouch, which has been used for blood procurement and flows through the filter into another pouch. The used filters employed in the method of the invention have preferably been used until their designated blood capacity has been reached. Typically filters are designated to reduce leukocytes from about 500 ml whole blood, which is the average size of a blood donation. Consequently, such a filter would be employed preferentially after 500 ml of blood, preferably whole blood have been passed through.

In a preferred embodiment of the method of present invention the leukocyte reduction filter comprises a polymer filter unit. In a preferred embodiment the polymer is polyacrylnitrile, polycarbonate, polyethylene (PE), polypropylene (PP), polytetrafluoroethylene (PTFE), polystyrene (PS), polysulfone, polyvinylchloride (PVC), cellulose acetate, polydimethylsiloxane, poly(ethylene terephtalate) (PET), poly(methyl methacrylate), polyurethane (PU), polyaramide, poly(hydroxyethylene methacrylate) polyvinylalcohol (PVA) and mixtures and/or copolymers thereof. More preferably the polymer is PE, PET, PP, PVA or PU and mixtures and/or copolymers thereof. A variety of filters comprising such polymers are commercially available and include, for example, Leukoflex LST-1 (Maco Pharma S.A., 200 Chaussée Fernand Forrest, 59200 Tourcoing, France), Compoflex® (Fresenius AG, Konzernzentrale, Else-Kröner-Str. 1, 61352 Bad Homburg, Germany), Sepacell® RZ-2000 (Baxter Deutschland GmbH, Fenwal Division, Edisonstr. 3 - 4 85716 München-Unterschleißheim), WBF-3 (Pall Corporation, 2200 Northern Boulevard East Hills, NY 11548) and Teruflex® WB-RP (Terumo Corporation, 2-44-1 Hatagaya, Shibuya-ku, Tokyo, 151-0072 Japan).

The term "back-flushing" refers to the application of fluid to the filter with a flow direction which is inversed with respect to the flow direction of the blood, which has been previously applied. The fluid can be applied by gravity or by negative or positive pressure. Positive pressure can, for example, be applied by a fluid filled syringe attached to the outlet port of the filter. Negative pressure can be applied, for example, by an empty syringe attached to the inlet port of the filter and withdrawing the piston, while the outlet port is attached to a fluid reservoir. The speed of back-flushing, should not be high, since this can lead to cell disruption. For a typical leukocyte reduction filter with a capacity for 500 ml blood back-flushing should be carried out at a flow rate of between about 0,5 to 5 ml/min and preferably of between about 1 to 2 ml/min. For a filter of a typical size, i.e. for 500 ml blood, a volume of about 100 ml to 200 ml buffer is sufficient for flushing out all desired cells.

The buffer used for the back-flushing comprises a chelating compound, preferably with chelating properties similar to EDTA, i.e. the ability to chelate divalent cations, more preferably EDTA or a salt thereof and a disaccharide preferably saccharose and can further comprise phosphate buffered saline as a buffering component. Other buffering components that are non toxic to cells can also be comprised within the buffer. The inclusion of a chelating compound in particular EDTA and of a disaccharide in particular saccharose leads on one hand to the efficient elution of a peripheral blood stem cell enriched cell fraction and increases on the other hand the viability and functionality of the stem cells, i.e. the pluripotency, of the obtained cell suspension. The cell suspension obtained with the process of the present invention can be stored after back-flushing for over 24 hours without detrimental effect to the cells. In a preferred embodiment the buffer comprises the chelating compound, preferably with chelating properties similar to EDTA, i.e. the ability to chelate divalent cations, more preferably EDTA or a salt thereof in the range of about 1-10 mmol/l and more preferably in the range of about 2 to 8 mmol/l and most preferably at a concentration of about 5 mmol/l and the disaccharide, preferably saccharose in the range of about 1 to 10% by weight, more preferably in the range of about 1.5 to 5% by weight and most preferably at a concentration of about 2.5% by weight.

The buffer can further comprise other agents like antimicrobial compounds, in particular antimycotic and antibacterial substances, stabilizing agents, in particular autologous plasma, human serum albumin or higher polysaccharides. In a preferred embodiment the buffer comprises autologous plasma from the recovered leukocyte containing filter. This plasma is added to the buffer in a preferred range of between 10 and 50%.

In another preferred embodiment the buffer consists of a chelating compound, preferably with chelating properties similar to EDTA, i.e. the ability to chelate divalent cations, more preferably EDTA or a salt thereof, saccharose and PBS. In a more preferred embodiment the buffer also includes autologous plasma. If the buffer consists of those components the concentrations are within the same ranges and preferred ranges that are indicated above. In a particularly preferred embodiment the buffer consists of PBS, 5 mmol/l EDTA, preferably in the form of EDTA-Na₂, and 2.5% by weight saccharose. PBS stands for phosphate buffered saline and comprises NaH₂PO₄ x H₂O and Na₂HPO₄ x 7 H₂O and NaCl. The phosphate concentration in the buffer varies in the range of 0.005 to 0.1 mol/l and the NaCl concentration varies in the range of 0.1 to 0.2 mol/l. The amount of phosphate in the buffer determines the buffer capacity of the phosphate buffered saline and a typical phosphate buffered saline comprises about 0.01 mol/l phosphate and about 0.15 mol/l NaCl. The pH is adjusted by H₃PO₄ or NaOH and typically is in the range of about pH 6.8 to pH 7.8, preferably in the range of about pH 7.2 to pH 7.4. In the more preferred embodiment autologous plasma from the recovered leukocyte containing filter derived blood donation is added to the buffer. Preferably in the range between 10 and 50%.

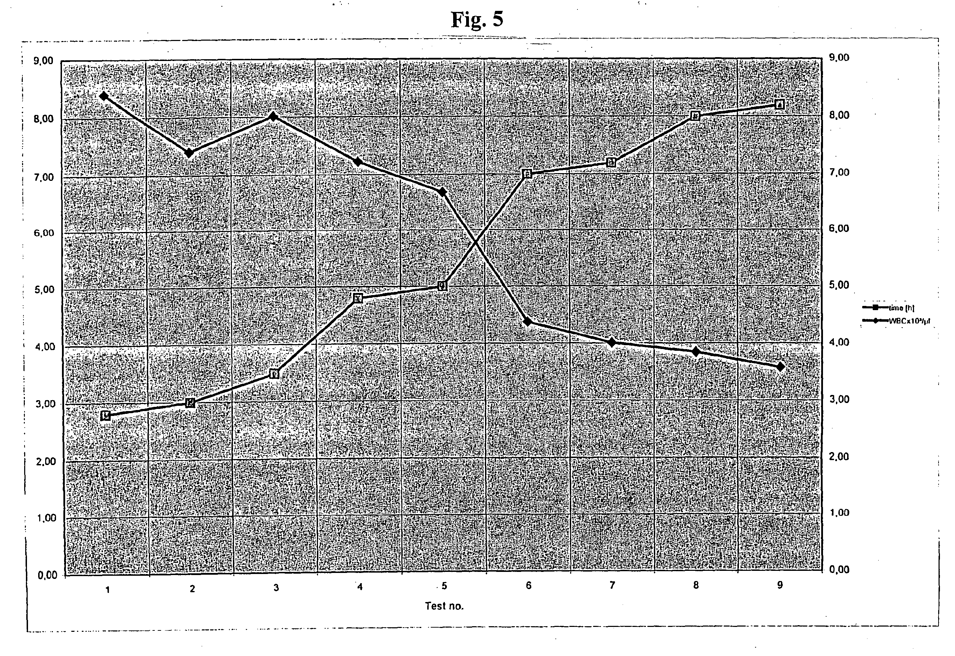

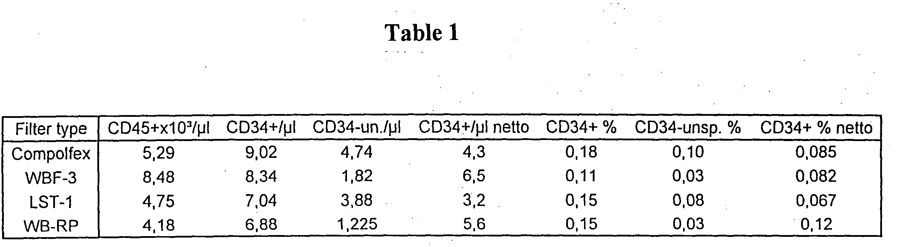

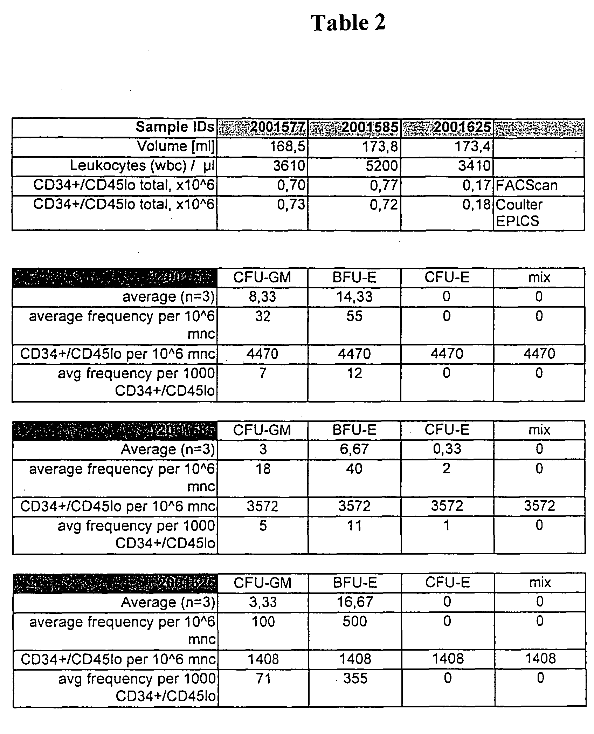

As outlined above the method of the present invention uses leukocyte reduction filters, which have previously been used for the depletion of blood, preferably of whole blood from a donor and which are, thus, filled with leukocytes. To obtain cells with optimal viability it is crucial that the back-flushing procedure is carried out within a short time after the filtration process of the blood has been completed, i.e. after the filter has been used as described above. Therefore, in a preferred embodiment of the method of the present invention the back-flushing of the leukocyte reduction filter is carried out less than about eight hours after the leukocyte reduction filter has been used. In a more preferred embodiment the back-flushing is carried out less than about six hours, more preferably less than about four hours, and most preferably less than about two hours after the leukocyte reduction filter has been used. When decreasing the time between the use of a leukocyte reduction filter and the back-flushing this not only leads to an increased viability of the cells but also to an increase in the recovery of mononuclear cells and stem cells, respectively (Fig. 5).

After the cells have been back-flushed the cells are recovered for further use. In its simplest form "recovering" means that the cells in the elution buffer are directly used in further processes or recovering entails (an) additional step(s) of concentration or purification which include, for example, sedimentation via gravity or centrifugation and/or immunodepletion. Sedimentation can be used to increase the concentration of the cells and/or to change the buffer or media in which the cells are contained. Routinely cells which are meant to be subcultured are sedimented by gentle centrifugation and then resuspended in an appropriate cell culture medium. However, centrifugation is also carried out at as means of further purification of the cell fraction enriched for peripheral blood stem cells using a density gradient centrifugation method. These gradients are well-known in the art and comprise, for example, Ficoll or Percoll centrifugation. Another method known to the skilled artisan is the carbonyl iron fractionation of peripheral blood progenitor cells utilizing magnetic sedimentation.

As outlined above peripheral blood stem cells are characterized by expressing the CD34⁺ cell surface receptor and, thus, can both be distinguished and separated from other cells based on this molecular marker. To that end antibodies reactive to the CD34⁺ surface marker can be used. Such methods are called immunoprecipitation or immunodepletion, since they use an antibody for the specific targeting of a certain subtype of cells within a larger cell population. Several approaches for immunodepletion are known in the art which comprise, for example, cross-linking of antibodies to beads or column surfaces and incubation of the eluted cells with the beads or application of a cell suspension to a column and, if desired subsequent elution of the cells from the column or the beads. Such additional separation steps can yield almost pure peripheral blood stem cells.

In a preferred embodiment the method of the present invention is carried out at a temperature between about 18 and about 22°C.

In view of the surprising finding of the present invention another aspect of the present invention is the use of a leukocyte reduction filter for the preparation of a cell suspension enriched for peripheral blood stem cells.

As outlined above the leukocyte cell suspension obtained when back-flushing a leukocyte reduction filter comprises at least 0.01% peripheral blood stem cells, preferably at least 0.02%, more preferably at least 0.03%, more preferably at least 0.04%, more preferably at least 0.05%, more preferably at least 0.06%, more preferably at least 0.07%, more preferably at least 0.08%, more preferably at least 0.09% and most preferably at least 0.1% CD34⁺ cells.

The following examples are illustrations of the invention and are not meant in any way to limit the scope to the invention, which is determined by the appended claims. Furthermore all references cited herein are incorporated herewith by reference.

### DESCRIPTION OF THE FIGURES

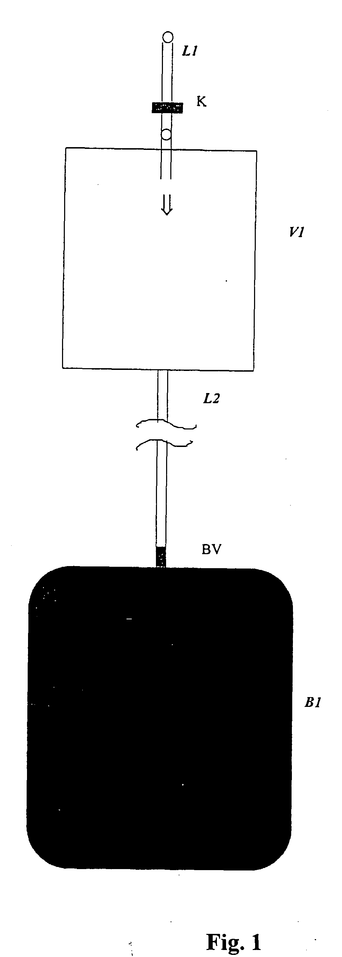

Fig 1: Drawing of a set up for back-flushing of a filter with a bag system without a bypass (part drawing of the set up). The size of the configuration shown is as follows: L1 = tubing upstream of filter, typically 29 cm to 33 cm and L2 = tubing downstream of filter, typically 31 cm to 46 cm; V1 = reduction filter volume of the filter, typically 30 ml to 100 ml; B1 = sterile receiving pouch (volume 450 ml to 500 ml); K = clamp and BV = breaking valve.

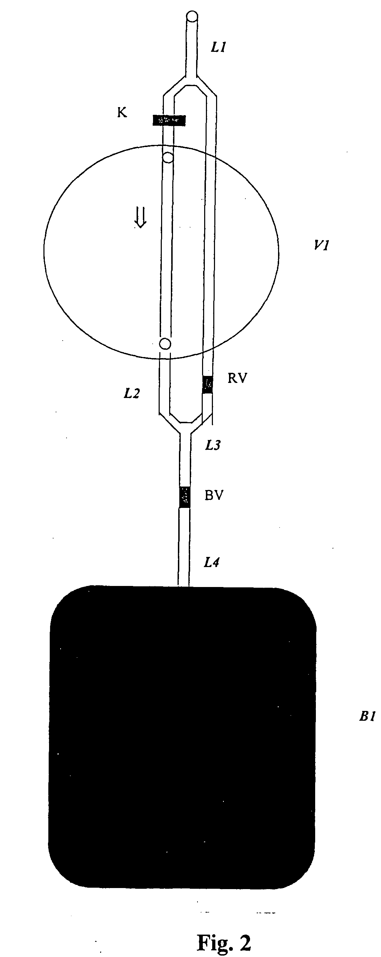

Fig. 2: Drawing of a set up for back-flushing of a filter system with a bypass (part drawing of the set up). The size of the configuration is as follows. L1 = 1 cm to 9 cm; L2 = 4 cm to 12 cm; L3 = 1 cm to 9 cm; L4 = 13 cm to 29 cm; V1 = reduction filter, volume of the filter V1 = 20 ml to 40 ml; B1 sterile receiving pouch volume ( volume 450 ml to 500 ml); K = clamp; RV = return valve and BV = breaking valve.

Fig 3: Yield of different leukocyte fractions from leukocyte depletion filters in percentage of the cell fraction.

Fig. 4: Recovery of CD45⁺ PBMC and CD34⁺ stem cells of leukocyte reduction filters. The yield from back-flushing using four different types of filters which have been used for depletion of whole blood is shown. The yield is given as percentage of the total cell number.

Fig. 5: Influence of time between leukocyte reduction and filter back-flushing. The yield is reduced if the back-flushing commences later than 4 hours after leukocyte depletion of whole blood.
Table 1: Shows numerical values for the results depicted in Fig. 4.
Table 2: Shows the results of the colony assay of CD34⁺ stem cells from leukocyte reduction filters out of three experiments

### EXAMPLES

### Elution of cells

### 1.1

Soft case filter (LST-1, WB-RP): The used filter (arrow on the case pointing upwards) is extended and the tubing is closed with a clamp. The tube is cut about 1 cm of the filter and a sterile 100 ml syringe is attached using a luer-lock. The clamp is released and 100 ml buffer (phosphate buffered saline 10 mmol/l phosphate, 0.015 mol/l NaCl, pH 7.2-7.4; diluted from a 10 fold stock solution comprising 5 mmol/l EDTA-Na₂ and 2.5% by weight saccharose (Biochemical grade) is applied to the filter. Then an additional 50 ml of buffer is applied to the filter and in case that elution is not commenced positive pressure can be applied with an air filled syringe. The elution is completed after the back side of the filter is empty and air has pushed eluate out of filter completely. Generally the procedure takes approximately three to ten minutes. The eluate flows into the sterile receiving pouch which is after completion of elution closed under sterile conditions.

### 1.2

Hard case filter (Compoflex, RZ 2000, WBF-3): The used filter (arrow on the case pointing up) is extended and the tubing is closed with a clamp. The tube is cut about 1 cm of the filter and a sterile 100 ml syringe is attached to the tube using a luer-lock. The clamp is released, the filter is degassed and 100 ml buffer, is slowly pressed through the filter at a flow speed of 0.5-2 ml/sec. A final volume of 50 ml buffer is passed through the filter in a second elution step. The elution immediately commences upon the filling of the filter with the buffer (10 mmol/l phosphate, 0.015 mol/l wall PBS including 5 mmol/l EDTA plus 2.5% by weight saccharose). In an additional step the remaining liquid is slowly pressed out of the filter by air pressure. The eluate is received in the sterile receiving pouch which is closed after completing the elution under sterile conditions.

### 2. Characterization of eluted cell fractions

### 2.1 Determination of leukocytes:

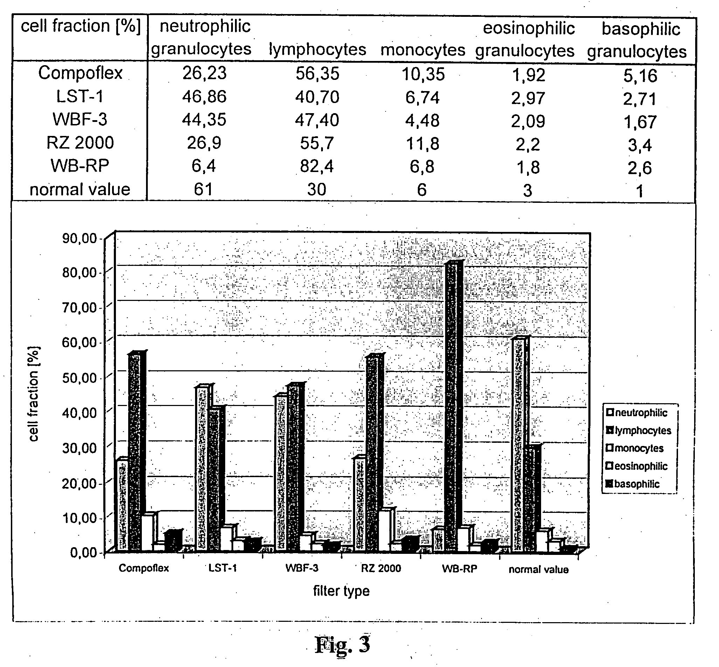

For the determination of the success of the elution initially a flow cytometric differentiation of the leukocytes was carried out by light scattering in form of a scattergram. This was carried out with a Cell-Dyn 3200 (Abbott Laboratories Incorporated, Abbott Park Road, North Chicago, IL 60064-3502 USA) according to the manufactory instructions. It allowed an analysis of the leukocyte fraction and a differentiation between neutrophils, lymphocytes, monocytes, eosinophils and basophils. The results are shown in Fig. 3.

### 2.2 Characterization of the mononuclear cell fraction:

For a further identification of the individual cell types the following test system was used and evaluated flow cytometrically with a FACSCalibur (Becton Dickinson Biosciences, Tullastr. 8 - 12, 69126 Heidelberg, Germany). All reagents were obtained from Becton Dickinson. This allowed a specific determination of the different cell subtypes and the differentiation between viable cells and cell fragments. The evaluation was carried out using the automated MultiTEST™ immunophenotyping software. The following surface markers were used to assign cells to the different cell lineages:
- Lymphocytes:: CD3⁺/CD45⁺
- T helper cells:: CD3⁺/CD4⁺/CD45⁺
- Suppressor cells:: CD3⁺/CD8⁺/CD45⁺
- B lymphocytes:: CD19⁺/CD45⁺
- NK-cells:: CD16⁺/CD56⁺/CD45⁺
- Monocytes:: CD14⁺/CD45⁺

### 2.3 FACS determination of CD34⁺ cells:

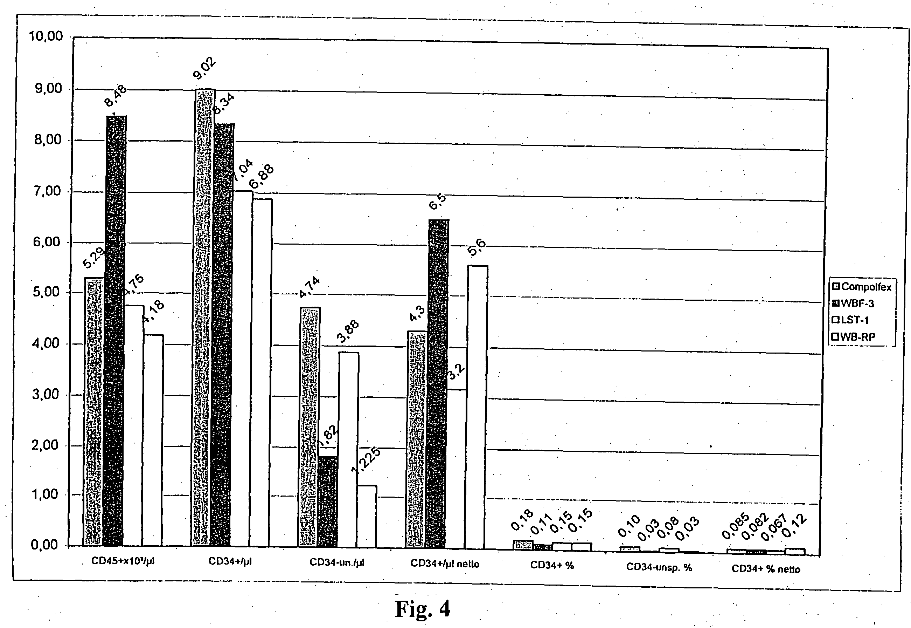

For the identification of the absolute count of CD34⁺ cells ProCOUNT™ Progenitor Cell Enumeration kit (Becton Dickinson) was used in combination with ProCOUNT™ phenotyping software on the FACSCalibur. In duplicate experiments one test tube was mixed with CD34 staining reagent and another set of test tubes with a control reagent. The staining reagent specifically stains stem cells and contains a nucleic acid staining dye a murin monoclonal anti-human CD34 antibody which is coupled to phycoerythrin and a PerCP-coupled monoclonal anti-human CD45 antibody (CD45 is the leukocyte common antigen). The control reagent contains anti-human CD45 antibody, a nucleic acid specific dye and an anti-IgG1 antibody as a marker for all cells other than stem cells. TruCOUNT™ Beads were used as an internal standard for assessing the correctness of the cell counting. The results of these experiments using three different types of used filters for back-flushing and an evaluation of the cell distribution within the recovered cell suspension is shown in Fig. 4. In addition the quantitation of this experiment is summarized in Table 1.

### 2.4 Colony forming assay:

The type of filters used are Compoflex, LST-1 and WBF-3. The gross recovery of CD34⁺ cells from these filters did not vary markedly, however, the WBF-3 filter showed significantly lower unspecific CD34⁺ binding, with the result that the WBF-3 filter showed a very good yield of CD34⁺ cells.

Cell culturing was used as a determination and quantification of the capability of the cells to form colonies of different lineages, which is a test of the functionality of the blood stem cells comprised within the cell fraction.

To this end, the cell suspension recovered from the leukocyte reduction filters were mixed with IMDM (= Iscove's modified Dulbecco's medium) (GibcoBRL, Division of Life Technologies Inc., P.O. Box 9418 Gaithersburg, MD 20898, USA) + 2% fetal clone III serum "FCS HyClone" (HyClone, 925 West 1800 South, Logan, UT 84321) and separated by centrifugation using Ficoll 1.077 (Biochrome AG, Berlin, Germany). Subsequently, the isolated mononuclear cells were washed with IMDM plus 2% FCS and resuspended in the same medium. The count of mononuclear cells was determined and the cells were inoculated in 0.3 ml tissue culture medium (IMDM + 2% FCS) in a 24 well tissue culture plate at densities of 5 x 10³, 1 x 10⁴ and 5 x 10⁴ cells per tissue culture well. The intervening rows of the tissue culture plate were only filled with distilled water to prevent cross-contamination. The cells were then placed in an incubator at 37°C and in an atmosphere containing 5% CO₂ and cultivated for 14 days. All colonies with at least 50 cells are counted as one colony. The following colony forms were differentiated:
- CFU-GM =: colony forming units - human granulocytes/macrophages
- CFU-E =: colony forming units - human erythroid colonies
- BFU-E =: burst forming units - human erythroid colonies
- CFU-GEMM =: pluripotent mixed colonies.

The results of this experiment is shown in Table 2. It was possible to show that the CD34+ cell containing cell suspension continued to proliferate in the colony forming assay and was capable to differentiate into a variety of different hemapoetic cell lineages proving the pluripotency of the isolated CD34⁺ cells.

## Claims

1. Method of preparing peripheral blood stem cells from a used leukocyte reduction filter comprising the following steps:
a) back-flushing a leukocyte reduction filter, which has been used for the preparation of leukocyte poor blood, with a buffer comprising a chelating compound, preferably EDTA and a disaccharide, preferably saccharose and
b) recovering the cells from the buffer.

2. Method according to claim 1, wherein the leukocyte reduction filter comprises a polymer filter unit.

3. Method according to claim 1 or 2, wherein the polymer is polyacrylnitrile, polycarbonate, polyethylene (PE), polypropylene (PP), polytetrafluoroethylene (PTFE), polystyrene (PS), polysulfone, polyvinylchloride (PVC), cellulose acetate, polydimethylsiloxane, poly(ethylene terephtalate) (PET), poly(methyl methacrylate), polyurethane (PU), polyaramide, poly(hydroxyethylene methacrylate) polyvinylalcohol (PVA) and mixtures or copolymers thereof.

4. Method according to one of claims 1 to 3, wherein the buffer further comprises phosphate buffered saline (PBS) and/or autologous plasma.

5. Method according to claim 4, wherein the buffer consists of EDTA, saccharose, PBS and autologous plasma.

6. Method according to one of claims 1 to 5, wherein the back-flushing of the leukocyte reduction filter is carried out less than 8 hours, after the leukocyte reduction filter has been used.

7. Method according to claim 6, wherein the back-flushing of the leukocyte reduction filter is carried out less than 4 hours, after the leukocyte reduction filter has been used.

8. Method according to one of claims 1 to 7, wherein the cells are recovered by sedimentation and/or immunodepletion.

9. Method according to claim 8, wherein the sedimentation is carried out using a Ficoll or Percoll gradient.

10. Method according to claim 8, wherein the immunodepletion is carried out with a stem cell specific antibody.

11. Method according to claim 10, wherein the antibody is an anti-CD34 antibody.

12. Method according to one of claims 1 to 11, wherein the back-flushing is carried out at temperatures of between 18 and 22 °C.

13. Use of a leukocyte reduction filter for the preparation of a cell suspension enriched for peripheral blood stem cells.

14. Use according to claim 13, wherein the cell suspension comprise at least 0,05% peripheral blood stem cells.
